(19) Europäisches Patentamt
European Patent Office
Office européen des brevets

(11) EP 2 171 074 B1

(12) # EUROPÄISCHE PATENTSCHRIFT

(45) Veröffentlichungstag und Bekanntmachung des Hinweises auf die Patenterteilung:
**30.11.2011 Patentblatt 2011/48**

(21) Anmeldenummer: **08774090.8**

(22) Anmeldetag: **16.06.2008**

(51) Int Cl.:
***C12P 7/02*** *(2006.01)*

(86) Internationale Anmeldenummer:
**PCT/EP2008/057522**

(87) Internationale Veröffentlichungsnummer:
**WO 2008/155302 (24.12.2008 Gazette 2008/52)**

(54) **Verfahren zur Herstellung optisch aktiver Alkohole unter Verwendung einer Dehydrogenase aus Azoarcus sp. EbN1**

Process for producing optically active alcohols employing an dehydrogenase derived from Azoarcus sp. EbN1

Procédé de production d'alcools optiquement actifs en utilisant d'une déshydrogénase dérivée de Azoarcus sp. EbN1

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **20.06.2007 EP 07110670**

(43) Veröffentlichungstag der Anmeldung:
**07.04.2010 Patentblatt 2010/14**

(73) Patentinhaber:
- **BASF SE**
  **67056 Ludwigshafen (DE)**
- **Albert-Ludwigs-Universität Freiburg**
  **79100 Freiburg (DE)**
- **Max-Planck-Gesellschaft zur Förderung der Wissenschaften e.V.**
  **80539 München (DE)**

(72) Erfinder:
- **BREUER, Michael**
  **64285 Darmstadt (DE)**
- **RABUS, Ralf**
  **26160 Bad Zwischenahn (DE)**
- **HEIDER, Johann**
  **35039 Marburg (DE)**

(56) Entgegenhaltungen:
**WO-A-2005/108590 WO-A-2006/094945**

- **DATABASE EMBL [Online] 29. Mai 2007 (2007-05-29), RABUS, R. ET AL.: "Short-chain dehydrogenase/reductase, possibly involved in polyhydroxybutyrate (PHB) synthesis" XP002500772 Database accession no. Q5P1L5 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 171 074 B1

SEQUENCE LISTING

<110> BASF SE und Max-Planck-Gesellschaft und Universität Freiburg

<120> Verfahren zur Herstellung optisch aktiver Alkohole

<130> PF 0000059328

<160> 8

<170> PatentIn version 3.1

<210> 1
<211> 741
<212> DNA
<213> Azoarcus sp. EbN1

<220>
<221> CDS
<222> (1)..(741)
<223>

<400> 1

```
atg aat cag aaa gtc gca ctc gtc acc ggc gcc atg ggt ggc ctg ggt       48
Met Asn Gln Lys Val Ala Leu Val Thr Gly Ala Met Gly Gly Leu Gly
1               5                   10                  15

acc gct atc tgc cag gcg ctg gca aag gac gga atg aag gtc gtg gcc       96
Thr Ala Ile Cys Gln Ala Leu Ala Lys Asp Gly Met Lys Val Val Ala
            20                  25                  30

aat tgt ctc ccc ggc ttt ccg cag aag gat gag tgg ctg gga cgg cag      144
Asn Cys Leu Pro Gly Phe Pro Gln Lys Asp Glu Trp Leu Gly Arg Gln
        35                  40                  45

aag gag ctc ggc ttc gat ttc atc gct gcc gaa ggc gac gta tcg gac      192
Lys Glu Leu Gly Phe Asp Phe Ile Ala Ala Glu Gly Asp Val Ser Asp
    50                  55                  60

tat gac tcc tgt cgc gcg atg gtg gcg aag atc gag ggc gag gtg ggt      240
Tyr Asp Ser Cys Arg Ala Met Val Ala Lys Ile Glu Gly Glu Val Gly
65                  70                  75                  80

gcg atc gat gtg ctg gtg aac aac gcc ggg atc acc cgc gac aag ttc      288
Ala Ile Asp Val Leu Val Asn Asn Ala Gly Ile Thr Arg Asp Lys Phe
                85                  90                  95

ttc ccg aag atg gaa aag gtg cag tgg gat gcg gtc atc aac acc aac      336
Phe Pro Lys Met Glu Lys Val Gln Trp Asp Ala Val Ile Asn Thr Asn
            100                 105                 110

ctc aac agc ctt ttc aac gtc act cac cac gtt tcg ccg aag atg gca      384
Leu Asn Ser Leu Phe Asn Val Thr His His Val Ser Pro Lys Met Ala
        115                 120                 125

gaa cgg ggc tat ggc cga atc atc aat att tct tcg gtg aac ggc gtc      432
Glu Arg Gly Tyr Gly Arg Ile Ile Asn Ile Ser Ser Val Asn Gly Val
    130                 135                 140

aag ggc cag gcc ggc cag acc aac tac tcg act gcc aag gcg ggc gtg      480
Lys Gly Gln Ala Gly Gln Thr Asn Tyr Ser Thr Ala Lys Ala Gly Val
145                 150                 155                 160

ctc ggc ttc acg aaa gcc ctt gcc gcg gaa ctg gcg acg aaa ggc gtg      528
Leu Gly Phe Thr Lys Ala Leu Ala Ala Glu Leu Ala Thr Lys Gly Val
                165                 170                 175

acc gtc aat gcg atc gcg ccg ggc tat atc ggc acc gag atg gtg atg      576
Thr Val Asn Ala Ile Ala Pro Gly Tyr Ile Gly Thr Glu Met Val Met
            180                 185                 190

gcg att cgc gaa gac att cgc cag ggc atc atc gac agc gtc ccg atg      624
Ala Ile Arg Glu Asp Ile Arg Gln Gly Ile Ile Asp Ser Val Pro Met
        195                 200                 205

aag cgc ctg ggc aag ccg gaa gaa atc ggc gct ctg tgc tcc tac ctg      672
Lys Arg Leu Gly Lys Pro Glu Glu Ile Gly Ala Leu Cys Ser Tyr Leu
    210                 215                 220

tct tcc gat ctg gcc ggt tac gtg acc ggc gcg acg atc aac atc aac      720
Ser Ser Asp Leu Ala Gly Tyr Val Thr Gly Ala Thr Ile Asn Ile Asn
225                 230                 235                 240

ggc ggc ctc cac atg tgc tga                                          741
Gly Gly Leu His Met Cys
                245
```

<210> 2
<211> 246
<212> PRT
<213> Azoarcus sp. EbN1

<400> 2

```
Met Asn Gln Lys Val Ala Leu Val Thr Gly Ala Met Gly Gly Leu Gly
1               5                   10                  15

Thr Ala Ile Cys Gln Ala Leu Ala Lys Asp Gly Met Lys Val Val Ala
            20                  25                  30

Asn Cys Leu Pro Gly Phe Pro Gln Lys Asp Glu Trp Leu Gly Arg Gln
        35                  40                  45

Lys Glu Leu Gly Phe Asp Phe Ile Ala Ala Glu Gly Asp Val Ser Asp
    50                  55                  60

Tyr Asp Ser Cys Arg Ala Met Val Ala Lys Ile Glu Gly Glu Val Gly
65                  70                  75                  80

Ala Ile Asp Val Leu Val Asn Asn Ala Gly Ile Thr Arg Asp Lys Phe
                85                  90                  95

Phe Pro Lys Met Glu Lys Val Gln Trp Asp Ala Val Ile Asn Thr Asn
```

```
            100                 105                 110

Leu Asn Ser Leu Phe Asn Val Thr His His Val Ser Pro Lys Met Ala
        115                 120                 125

Glu Arg Gly Tyr Gly Arg Ile Ile Asn Ile Ser Ser Val Asn Gly Val
    130                 135                 140

Lys Gly Gln Ala Gly Gln Thr Asn Tyr Ser Thr Ala Lys Ala Gly Val
145                 150                 155                 160

Leu Gly Phe Thr Lys Ala Leu Ala Ala Glu Leu Ala Thr Lys Gly Val
                165                 170                 175

Thr Val Asn Ala Ile Ala Pro Gly Tyr Ile Gly Thr Glu Met Val Met
            180                 185                 190

Ala Ile Arg Glu Asp Ile Arg Gln Gly Ile Ile Asp Ser Val Pro Met
        195                 200                 205

Lys Arg Leu Gly Lys Pro Glu Glu Ile Gly Ala Leu Cys Ser Tyr Leu
    210                 215                 220

Ser Ser Asp Leu Ala Gly Tyr Val Thr Gly Ala Thr Ile Asn Ile Asn
225                 230                 235                 240

Gly Gly Leu His Met Cys
                245
```

<210> 3
<211> 747
<212> DNA
<213> Azoarcus sp. EbN1

<220>
<221> CDS
<222> (1)..(747)
<223>

<400> 3

```
atg ctg ctc gaa ggg aaa acc gcg ctg gtg acg ggt gcc ggc aac ggc       48
Met Leu Leu Glu Gly Lys Thr Ala Leu Val Thr Gly Ala Gly Asn Gly
1               5                   10                  15

atc ggc cgc acc atc gcg ctc acc tac gcc gcc gaa ggg gcg aac gtc       96
Ile Gly Arg Thr Ile Ala Leu Thr Tyr Ala Ala Glu Gly Ala Asn Val
            20                  25                  30

gtc gtt tcc gac atc agt gac gaa tgg ggc cgg gaa aca ctc gcc ctg      144
Val Val Ser Asp Ile Ser Asp Glu Trp Gly Arg Glu Thr Leu Ala Leu
```

```
            35                  40                  45

        atc gaa ggc aag ggc gga aaa gcc gtt ttc caa cac gcc gac acc gcc      192
        Ile Glu Gly Lys Gly Gly Lys Ala Val Phe Gln His Ala Asp Thr Ala
            50                  55                  60

        cac ccc gaa gac cat gac gag ctg atc gcc gcg gcc aaa cgc gcc ttc      240
        His Pro Glu Asp His Asp Glu Leu Ile Ala Ala Ala Lys Arg Ala Phe
        65                  70                  75                  80

        ggc cgc ctc gac att gcc tgc aac aac gcc ggc atc agc ggc gaa ttc      288
        Gly Arg Leu Asp Ile Ala Cys Asn Asn Ala Gly Ile Ser Gly Glu Phe
                        85                  90                  95

        acc cct acc gcg gaa acg acc gac gcc cag tgg caa cga gtc atc ggc      336
        Thr Pro Thr Ala Glu Thr Thr Asp Ala Gln Trp Gln Arg Val Ile Gly
                    100                 105                 110

        atc aac ctg tcg ggc gtg ttc tac ggc gtg cgt gcg cag att cgc gcc      384
        Ile Asn Leu Ser Gly Val Phe Tyr Gly Val Arg Ala Gln Ile Arg Ala
                115                 120                 125

        atg ctc gaa acc gga ggc ggc gcg atc gtc aat att tct tcc att gcc      432
        Met Leu Glu Thr Gly Gly Gly Ala Ile Val Asn Ile Ser Ser Ile Ala
            130                 135                 140

        ggg cag atc ggc atc gag ggc atc acg ccc tac acc gcc gcc aag cac      480
        Gly Gln Ile Gly Ile Glu Gly Ile Thr Pro Tyr Thr Ala Ala Lys His
        145                 150                 155                 160

        ggc gtg gtg ggt ctg acg aaa acg gtc gcc tgg gaa tat ggc agc aag      528
        Gly Val Val Gly Leu Thr Lys Thr Val Ala Trp Glu Tyr Gly Ser Lys
                        165                 170                 175

        ggc atc cgc atc aat tcg gtc ggt ccg gcc ttc atc aat acc acg ctg      576
        Gly Ile Arg Ile Asn Ser Val Gly Pro Ala Phe Ile Asn Thr Thr Leu
                    180                 185                 190

        gtt cag aac gtt ccc ctc gaa aca cgc cgg cag ctc gaa cag atg cac      624
        Val Gln Asn Val Pro Leu Glu Thr Arg Arg Gln Leu Glu Gln Met His
                195                 200                 205

        gcc ctg cgc cgc cta ggc gaa acg gaa gaa gtc gcc aat ctc gtc gcc      672
        Ala Leu Arg Arg Leu Gly Glu Thr Glu Glu Val Ala Asn Leu Val Ala
            210                 215                 220

        tgg ctg agc agc gac aag gcc agc ttc gtc acc ggc agc tat tac gcg      720
        Trp Leu Ser Ser Asp Lys Ala Ser Phe Val Thr Gly Ser Tyr Tyr Ala
        225                 230                 235                 240

        gtc gac ggc ggc tac ctc gca cga tga                                  747
        Val Asp Gly Gly Tyr Leu Ala Arg
                        245
```

<210> 4
<211> 248
<212> PRT
<213> Azoarcus sp. EbN1

<400> 4

```
Met Leu Leu Glu Gly Lys Thr Ala Leu Val Thr Gly Ala Gly Asn Gly
1               5                   10                  15

Ile Gly Arg Thr Ile Ala Leu Thr Tyr Ala Ala Glu Gly Ala Asn Val
            20                  25                  30

Val Val Ser Asp Ile Ser Asp Glu Trp Gly Arg Glu Thr Leu Ala Leu
        35                  40                  45

Ile Glu Gly Lys Gly Gly Lys Ala Val Phe Gln His Ala Asp Thr Ala
    50                  55                  60

His Pro Glu Asp His Asp Glu Leu Ile Ala Ala Ala Lys Arg Ala Phe
65                  70                  75                  80

Gly Arg Leu Asp Ile Ala Cys Asn Asn Ala Gly Ile Ser Gly Glu Phe
                85                  90                  95

Thr Pro Thr Ala Glu Thr Thr Asp Ala Gln Trp Gln Arg Val Ile Gly
            100                 105                 110

Ile Asn Leu Ser Gly Val Phe Tyr Gly Val Arg Ala Gln Ile Arg Ala
        115                 120                 125

Met Leu Glu Thr Gly Gly Gly Ala Ile Val Asn Ile Ser Ser Ile Ala
    130                 135                 140

Gly Gln Ile Gly Ile Glu Gly Ile Thr Pro Tyr Thr Ala Ala Lys His
145                 150                 155                 160

Gly Val Val Gly Leu Thr Lys Thr Val Ala Trp Glu Tyr Gly Ser Lys
                165                 170                 175

Gly Ile Arg Ile Asn Ser Val Gly Pro Ala Phe Ile Asn Thr Thr Leu
            180                 185                 190

Val Gln Asn Val Pro Leu Glu Thr Arg Arg Gln Leu Glu Gln Met His
        195                 200                 205

Ala Leu Arg Arg Leu Gly Glu Thr Glu Glu Val Ala Asn Leu Val Ala
    210                 215                 220

Trp Leu Ser Ser Asp Lys Ala Ser Phe Val Thr Gly Ser Tyr Tyr Ala
225                 230                 235                 240

Val Asp Gly Gly Tyr Leu Ala Arg
                245
```

<210> 5
<211> 30
<212> DNA
<213> Artificial Sequence

<220>
<223> PCR-Primer

<400> 5
gcgattgcat atgaatcaga aagtcgcact 30

<210> 6
<211> 32
<212> DNA
<213> Artificial Sequence

<220>
<223> PCR-Primer

<400> 6
gcgcaggctt cggatcctgc atcagcacat gt 32

<210> 7
<211> 30
<212> DNA
<213> Artificial Sequence

<220>
<223> PCR-Primer

<400> 7
gcgattgcat atgctgctcg aagggaaaac 30

<210> 8
<211> 30
<212> DNA
<213> Artificial Sequence

<220>
<223> PCR-Primer

<400> 8
ctgatagatc ttagtgagcg atgaggatca 30

**Beschreibung**

Die vorliegende Erfindung betrifft ein Verfahren zur Herstellung von optisch aktiven Alkoholen der Formel Ia oder Ib

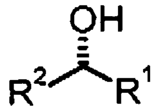
Formel Ia

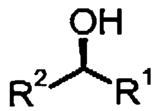
Formel Ib

Stand der Technik

Die Funktion von Dehydrogenasen als Biokatalysatoren ist allgemein bekannt [Chemico-Biological Interactions (2003) 143:247, Journal of Biological Chemistry (2002) 277:25677]. Insbesondere die technische Anwendung dieser Enzymklasse zur Herstellung von Feinchernikalien ist dokumentiert [Tetrahedron (2004) 60:633, Trends Biotechnol (1999) 17:487]. Je nach Substrat unterscheiden sich die bekannten Dehydrogenase in ihrer Aktivität und Spezifität. Man unterscheider bezüglich sie bezüglich Ihrer Stereoselektivität in sogenannte 'Prelog'- und 'anti'-Prelog-Enzyme (Pure and Applied Chemistry, (1964), 9:119).
So sind zur Herstellung von optisch aktiven Phenylethanolderivaten vornehmlich solche Biokatalysatoren beschrieben, die 'Prelog-Selektiviät aufweisen, Enzyme, die die gegensinnige Enantioselektivität aufweisen, sind seltener, wenn auch nicht unbekannt [Trends Biotechnol (1999) 17:487, J.Org.Chem. (1992) 57:1532]
WO 2005/108590 beschreibt ein Verfahren zur Herstellung von optisch aktiven Alkanolen aus den entsprechenden Alkanonen durch enzymatische Reduktion, wobei die im Laufe der Reaktion verbrauchten Reduktionsäquivalente durch Umsetzen eines Opfer-Alkohols zum Opfer_Keton wieder regeneriert werden.
WO 2006/094945 beschreibt ein Verfahren zur Herstellung von optisch aktiven Alkanolen spezieller Struktur durch Reduktion der entsprechenden Ketone mittels einer Dehydrogenase aus einem Organismus der Gattung Azoarcus.
Die vorliegende Erfindung betrifft ein Verfahren zur Herstellung von optisch aktiven Alkoholen der Formel Ia oder Ib

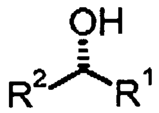
Formel Ia

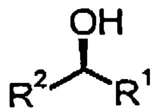
Formel Ib

worin

> $R^1$,$R^2$ für Alkyl, Alkenyl, Aryl, oderAlkylarylgruppen stehen, die wiederum ein- oder mehrfach substituiert sein können, durch Alkyl, Halogen, SH,$SR^2$ ,OH, $OR^2$, $NO_2$, CN, CO, $COOR^2$, $NR^2R^3$ oder $NR^2R^3R^{4+}X$, wobei $R^2$, $R^3$ und $R^4$ unabhängig voneinander für H oder einen Niedrigalkyl- oder Niedrigalkoxy-Rest stehen und X' für ein Gegenion steht, mit der Maßgabe, dass $R^1$ ungleich $R^2$ ist,

durch Reduktion des entsprechenden Ketons, wobei die Reduktion mit einer Dehydrogenase mit der Polypeptidsequenz SEQ ID NO:2, oder mit einer Polypeptidsequenz, bei der bis zu 25% der Aminosäurereste gegenüber SEQ ID NO:2 durch Deletion, Insertion, Substitution oder einer Kombination davon verändert sind, durchgeführt wird.
Eine besonders gute Ausführungsform der Erfindung besteht in einem Verfahren zur Herstellung von optisch aktiven Alkoholen der Formel Ia oder Ib, worin $R^1$, für C1-C10-Alkyl und $R^2$ für Phenyl, wobei die Reste R1 und/oder R2 ggf. durch Halogen einfach substituiert sind.
Die vorliegende Erfindung betrifft insbesondere ein Verfahren zur Herstellung optisch aktiver Alkohole der Formel Ia wobei für $R^1$ gilt, dass dieser Rest weniger raumerfüllend ist als $R^2$ .

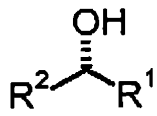

Formel Ia

Ist der Rest $R^2$ raumerfüllender als der Rest $R^1$, wird der Alkohol nach Prelog, V., Pure and Applied Chemistry, (1964), 9, 119-130 in die'anti'-Prelog-Kategorie einsortiert.

Chirale Alkohole können anhand Ihrer Konfiguration in sogenannte'Prelog' und'Anti-Prelog'-Enantiomere unterschieden werden. Die Zuordnung in eine der beiden Kategorien geschieht nach der Größe (Raumerfüllung) der beiden Gruppen, die der Alkoholgruppe benachbart sind und der Ausrichtung der Hydroxyfunktion in Bezug auf diese beiden Gruppen. Optisch aktive Alkohole mit'anti-Prelog' Konfiguration sind wichtige Vorstufen für verschiedene Wirkstoffe.

Allgemeine Begriffe und Definitionen

Werden keine andere Angaben gemacht, so gelten folgende allgemeine Bedeutungen:

"Halogen" steht für Fluor, Chlor, Brom oder Jod , insbesondere Fluor oder Chlor.

"Niedrigalkyl" steht für geradkettige oder verzweigte Alkylreste 1 bis 6 C-Atomen, wie Methyl, Ethyl, i- oder n-Propyl, n-, i-, sec.- oder tert.-Butyl, n-Pentyl oder 2-Methyl-Butyl, n-Hexyl, 2-Methyl-pentyl, 3-Methyl-pently, 2-Ethyl-butyl.

"Niedrigalkenyl" steht für die ein- oder mehrfach, vorzugsweise einfach oder zweifach ungesättigten Analoga oben genannter Alkylreste mit 2 bis 6 Kohlenstoffatomen, wobei die Doppelbindung in beliebiger Position der Kohlenstoffkette liegen kann.

"Niedrigalkoxy" steht für die Sauerstoff-terminierten Analoga obiger Alkylreste.

"Aryl" steht für einen ein- oder mehrkernigen, vorzugsweise ein- oder zweikernigen, gegebenenfalls substituierten aromatischen Rest, insbesondere für Phenyl oder für ein über eine beliebige Ringposition gebundenes Naphthyl, wie 1- oder 2-Naphthyl. Diese Arylreste können gegebenenfalls 1 oder 2 gleiche oder verschiedene Substituenten tragen, beispielsweise Halogen, Niedrigalkyl, Niedrigalkoxy gemäß obiger Definition oder Trifluormethyl.

"Enantioselektivität" im Rahmen der vorliegenden Erfindung bedeutet, dass der Enantiomerenüberschuss ee (in %) eines der beiden möglichen Enantiomere, wenigstens 50 %, vorzugsweise wenigstens 80 %, insbesondere wenigstens 90 % und speziell wenigstens 95 % beträgt. Der ee-Wert berechnet sich nach:

$$\text{ee (\%)} = \text{Enantiomer A} - \text{Enantiomer B} / (\text{Enantiomer A} + \text{Enantiomer B}) \times 100$$

Biochemische Ausführungsformen

Beschriebene Dehydrogenasen (EC 1.1.X.X) sind NAD- bzw. NADP-abhängige Dehydrogenasen (E.C. 1.1.1.x) insbesondere Alkoholdehydrogenasen (E.C.1.1.1.1 oder E.C.1.1.1.2), welche die selektive Reduktion des Ketons zum '*anti-Prelog*'-Alkohol bewirken. Die Dehydrogenase wird aus einem Mikroorganismus, besonders aus einem Bakterium, einem Pilz, insbesondere einer Hefe, jeweils hinterlegt in Stammsammlungen oder erhältlich aus Isolaten natürlicher Quelle, wie Bodenproben, Biomasseproben und dergleichen oder durch de novo-Gensynthese gewonnen.

Die Dehydrogenase kann in gereinigter oder teilweise gereinigter Form oder in Form des ursprünglichen Mikroorganismus oder eines rekombinanten Wirtsorganismus, der die Dehydrogenase exprimiert, verwendet werden. Verfahren zur Gewinnung und Aufreinigung von Dehydrogenasen aus Mikroorganismen sind dem Fachmann hinreichend bekannt, z.B- aus K. Nakamura & T. Matsuda, "Reduction of Ketones" in K. Drauz und H. Waldmann, Enzyme Catalysis in Organic Synthesis 2002, Vol.III, 991-1032, Wiley-VCH, Weinheim. Rekombinante Verfahren zur Erzeugung von Dehydrogenasen sind ebenfalls bekannt, beispielsweise aus W. Hummel, K. Abokitse, K. Drauz, C. Rollmann und H. Gröger, Adv. Synth. Catal. 2003, 345, Nr. 1 + 2, S. 153-159.

Geeignete Bakterien sind beispielsweise solche der Ordnungen der *Burkholderiales, Hydrogenophilales, Me-*

*thylophilales, Neisseriales, Nitrosomonadales, Procabacteriales* oder *Rhodocyclales.*

Beschrieben besonders werden Dehydrogenasen aus der Familie der Familie der *Rhodocyclaceae*.

Beschrieben werden werden Dehydrogenasen aus den Gattungen *Azoarcus Azonexus, Azospira, Azovibrio, Dechloromonas, Ferribacterium, Petrobacter, Propionivibrio, Quadricoccus, Rhodocyclus, Sterolibacterium, Thauera und Zoogloea.*

Beschrieben werden Dehydrogenasen aus Arten der Gattungen *Azoarcus.*

Üblicherweise erfolgt die Reduktion mit der Dehydrogenase in Gegenwart eines geeigneten Kofaktors (auch als Kosubstrat bezeichnet). Als Kofaktors für die Reduktion des Ketons dient üblicherweise NADH und/oder NADPH. Daneben können Dehydrogenasen als zelluläre Systeme eingesetzt werden, die inherent Kofaktor enthalten, oder alternative Redoxmediatoren zugesetzt werden (A. Schmidt, F. Hollmann und B. Bühler "Oxidation of Alcohols" in K. Drauz und H. Waldmann, Enzyme Catalysis in Organic Synthesis 2002, Vol.III, 991-1032, Wiley-VCH, Weinheim).

Üblicherweise erfolgt die Reduktion mit der Dehydrogenase außerdem in Gegenwart eines geeigneten Reduktionsmittels, welches den im Verlauf der Reduktion oxidierten Kofaktor regeneriert. Beispiele für geeignete Reduktionsmittels sind Zucker, insbesondere die Hexosen, wie Glukose, Mannose, Fructose, und/oder oxidierbare Alkohole, insbesondere Ethanol, Propanol, Butanol, Pentanol oder Isopropanol, sowie Formiat, Phosphit oder molekularer Wasserstoff. Zur Oxidation des Reduktionsmittels und damit verbunden zur Regeneration des Koenzyms kann eine zweite Dehydrogenase, wie z.B. Glukosedehydrogenase bei Verwendung von Glukose als Reduktionsmittel, Phosphitdehydrogenase bei Verwendung von Phosphit als Reduktionsmittel oder Formiat-Dehydrogenase bei der Verwendung von Formiat als Reduktionsmittel, zugesetzt werden. Diese kann als freies oder immobilisiertes Enzym oder in Form von freien oder immobilisierten Zellen eingesetzt werden. Ihre Herstellung kann sowohl separat als auch durch Koexpression in einem (rekombinanten) Dehydrogenase-Stamm erfolgen.

Die erfindungsgemäß verwendeten Dehydrogenasen können frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" in K. Drauz und H. Waldmann, Enzyme Catalysis in Organic Synthesis 2002, Vol. III, 991-1032, Wiley-VCH, Weinheim beschrieben.

Die Umsetzung kann in wässrigen oder nichtwässrigen Reaktionsmedien oder in 2-Phasensystemen oder (Mikro-)Emulsionen erfolgen. Bei den wässrigen Reaktionsmedien handelt es sich vorzugsweise um gepufferte Lösungen, die in der Regel einen pH-Wert von 4 bis 8, vorzugsweise von 5 bis 8, aufweisen. Das wässrige Lösungsmittel kann neben Wasser außerdem wenigstens einen Alkohol, z.B. Ethanol oder Isopropanol oder Dimethylsulfoxid enthalten.

Unter nicht-wässrigen Reaktionsmedien werden Reaktionsmedien verstanden, die weniger als 1 Gew.-%, vorzugsweise weniger als 0,5 Gew.-% Wasser, bezogen auf das Gesamtgewicht des Reaktionsmediums, enthalten. Vorzugsweise wird die Umsetzung in einem organischen Lösungsmittel durchgeführt. Geeignete Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon.

Beispielsweise wird die Reduktion mit der Dehydrogenase in einem wässrig-organischen, insbesondere wässrigen Reaktionsmedium durchgeführt.

Das zu reduzierende Keton wird vorzugsweise in einer Konzentration von 0,1 g/l bis 500 g/l, besonders bevorzugt von 1 g/l bis 50 g/l in die enzymatische Reduktion eingesetzt und kann kontinuierlich oder diskontinuierlich nachgeführt werden.

Die enzymatische Reduktion erfolgt in der Regel bei einer Reaktionstemperatur unterhalb der Desaktivierungstemperatur der eingesetzten Dehydrogenase und vorzugsweise bei wenigstens -10 °C. Besonders bevorzugt liegt sie im Bereich von 0 bis 100°C, insbesondere von 15 bis 60°C und speziell von 20 bis 40 °C, z.B. bei etwa 30 °C.

Zur Durchführung kann man beispielsweise das Keton mit der Dehydrogenase, dem Lösungsmittel und gegebenenfalls den Koenzymen, gegebenenfalls einer zweiten Dehydrogenase zur Regenerierung des Koenzyms und/oder weiteren Reduktionsmitteln vorlegen und das Gemisch durchmischen, z. B. durch Rühren oder Schütteln- Es ist aber auch möglich, die Dehydrogenase(n) in einem Reaktor, beispielsweise in einer Säule, zu immobilisieren, und durch den Reaktor eine das Keton und gegebenenfalls Koenzyme und/oder Kosubstrate enthaltende Mischung zu leiten. Hierzu kann man die Mischung im Kreislauf durch den Reaktor leiten bis der gewünschte Umsatz erreicht ist. Dabei wird die Ketogruppe des Ketons zu einer OH-Gruppe reduziert, wobei im Wesentlichen eines der beiden Enantiomere des Alkohols entsteht. In der Regel wird man die Reduktion bis zu einem Umsatz von wenigstens 70 %, besonders bevorzugt von wenigstens 85 % und insbesondere von wenigstens 95%, bezogen auf das in der Mischung enthaltene Keton führen. Das Fortschreiten der Reaktion, d. h. die sequentielle Reduktion des Ketons, kann dabei durch übliche Methoden wie Gaschromatographie oder Hochdruckflüssigkeitschromatographie verfolgt werden.

Besonders bevorzugt werden als Dehydrogenasen im erfindungsgemäßen Verfahren Alkohol-Dehydrogenasen mit folgenden Eigenschaften eingesetzt:

Alkoholdehydrogenase aus *Azoarcus* mit einer Aminosäuresequenz, gemäß Seq ID 2 sowie Alkoholdehydrogenasen mit Aminosäuresequenzen, bei denen bis zu 25% , bevorzugt bis zu 15%, besonders bevorzugt bis zu 10, insbesondere bis zu 5% der Aminosäurereste gegenüber SEQ ID NO:2 durch Deletion, Insertion, Substitution oder einer Kombination davon verändert sind.

Oxidation einfacher Alkohole wie zum Beispiel iso-Propanol, Butan-2-ol, Pentan-2-ol oder Cyclohexanol zum entsprechenden Carbonyl mit gleichzeitiger Reduktion von $NAD^+$ oder $NADP^+$.

Alkohol-Dehydrogenasen, welche die Reduktion in einer Enantiomerenreinheit von wenigstens 95 % ee (in Gegenwart von NADH und/oder NADPH; bei 30°C und pH 7.0) katalysiert.

Ein weiterer Gegenstand der vorliegenden Beschreibung auch eine '*anti-Prelog*'-Dehydrogenase mit wenigstens einer der vorstehend aufgeführten Eigenschaften.

Die Alkoholdehydrogenasen weisen Akivität in Gegenwart der folgenden Lösungsmitel auf: Heptan, Hexan, MtBE, n-Butanol, Butan-2-ol, n-Pentanol, Pentan-2-ol, Pentan-3-ol, DMSO *i*-Propanol,
n-Propanol, Ethanol.

Bevorzugt besitzen sie ein Molekulargewicht im Bereich von 26 $\pm$ 2 kDalton.

Weitere Abwandlungen der Dehydrogenasen:

Beschrieben sind ebenfalls "funktionale Äquivalente" der konkret offenbarten Enzyme mit Dehydrogenase-Aktivität und die Verwendung dieser in den erfindungsgemäßen Verfahren.

"Funktionale Äquivalente" oder Analoga der konkret offenbarten Enzyme sind davon verschiedene Polypeptide, welche weiterhin die gewünschte biologische Aktivität, wie z.B. Substratspezifität, besitzen. So versteht man beispielsweise unter "funktionalen Äquivalenten" Enzyme, die vom Keton zum entsprechenden 'anti-Prelog'-Alkohol reduzieren und die mindestens 20 %, bevorzugt 50 %, besonders bevorzugt 75 %, ganz besonders bevorzugt 90 % der Aktivität eines Enzyms, umfassend eine der unter Seq ID 2 aufgeführten Aminosäuresequenzen, aufweist. Funktionale Äquivalente sind außerdem vorzugsweise zwischen pH 4 bis 10 stabil und besitzen vorteilhaft ein pH-Optimum zwischen pH 5 und 8 sowie ein Temperaturoptimum im Bereich von 20°C bis 80°C.

Unter "funktionalen Äquivalenten" versteht man insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Beispiele für geeignete Aminosäuresubstitutionen sind folgender Tabelle zu entnehmen:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |

(fortgesetzt)

| Ursprünglicher Rest | Beispiele der Substitution |
|---|---|
| Glu | Asp |
| Gly | Pro |
| His | Asn ; Gln |
| Ile | Leu; Val |
| Leu | Ile, Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie funktionale Derivate" und "Salze" der Polypeptide.

"Präkursoren" sind dabei natürliche oder synthetische Vorstufen der Polypeptide mit oder ohne der gewünschten biologischen Aktivität.

Unter dem Ausdruck "Salze" versteht man sowohl Salze von Carboxylgruppen als auch Säureadditionssalze von Aminogruppen der Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin. Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Beschreibung.

"Funktionale Derivate" der Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

Im Falle einer möglichen Proteinglykosylierung umfassen "funktionale Äquivalente" Proteine des oben bezeichneten Typs in deglykosylierter bzw. glykosylierter Form sowie durch Veränderung des Glykosylierungsmusters erhältliche abgewandelte Formen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Beschreibung äquivalente Enzyme ermitteln.

"Funktionale Äquivalente" umfassen ebenfalls Fragmente, vorzugsweise einzelne Domänen oder Sequenzmotive, der Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide oder Enzyme.

Beschriebene, funktionale Äquivalente" sind Homologe zu den konkret offenbarten Proteinen. Diese besitzen wenigstens 75% ins besondere wenigsten 85 %, wie z.B. 90%, 95%, 97% oder 99%, Homologie zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie eines homologen Polypeptids bedeutet insbesondere prozentuale Identität der Aminosäurereste bezogen auf die Gesamtlänge einer der hierin konkret beschriebenen Aminosäuresequenzen.

Homologe der Proteine oder Polypeptide können durch Mutagenese erzeugt werden, z.B. durch Punktmutation oder Verkürzung des Proteins.

Homologe der Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese Beschriebener Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Weitere Ausgestaltung kodierender Nukleinsäuresequenzen

Gegenstand der Beschreibung sind Verwendung von Nukleinsäuresequenzen (einzel- und doppelsträngige DNA- und RNA-Sequenzen, wie z.B. cDNA und mRNA), die für ein Enzym mit Dehydrogenase-Aktivität kodieren. Bevorzugt sind Nukleinsäuresequenzen, welche z.B. für Aminosäuresequenzen gemäß Seq ID 2 oder charakteristische Teilsequenzen davon kodieren, oder Nukleinsäuresequenzen gemäß Seq ID 1 bzw. oder charakteristische Teilsequenzen davon umfassen.

Alle hierin erwähnten Nukleinsäuresequenzen sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet. 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Beschreibung sind auch Nukleinsäuresequenzen (einzel- und doppelsträngige DNA- und EtNA-Sequenzen, wie z.B. cDNA und mRNA), kodierend für eines der obigen Polypeptide und deren funktionalen Äquivalenten, welche z.B. unter Verwendung künstlicher Nukleotidanaloga zugänglich sind.

Die Beschreibung betrifft sowohl isolierte Nukleinsäuremoleküle, welche für Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifikation von kodierenden Nukleinsäuren verwendet werden können.

Die Nukleinsäuremoleküle können zudem untranslatierte Sequenzen vom 3'- und/oder 5'-Ende des kodierenden Genbereichs enthalten

Die Beschreibung umfasst weiterhin die zu den konkret beschriebenen Nukleotidsequenzen komplementären Nukleinsäuremoleküle oder einen Abschnitt davon.

Die Nukleotidsequenzen ermöglichen die Erzeugung von Sonden und Primern, die zur Identifizierung und/oder Klonierung von homologer Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein Nukleinsäuremolekül kann mittels molekularbiologischer Standard-Techniken und der bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor,

NY, 1989) verwendet werden. Überdies lässt sich ein NukleinsäuremoleKül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die Oligonukleotide können ferner durch Standard-Syntheseverfahren, z.B. mit einem automatischen DNA-Synthesegerät, hergestellt werden.

Die Nukleinsäuresequenzen lassen sich prinzipiell aus allen Organismen identifizieren und isolieren. Vorteilhaft lassen sich die Nukleinsäuresequenzen oder die Homologen davon, aus Pilzen, Hefen, Archeen oder Bakterien isolieren. Als Bakterien seien gram-negative und gram-positive Bakterien genannt. Bevorzugt werden die Nukleinsäuren aus gram-negativen Bakterien vorteilhaft aus $\alpha$-Proteobakterien, $\beta$-Proteobakterien oder y-Proteobakterien, besonders bevorzugt aus Bakterien der Ordnungen der *Burkholderiales, Hydrogenaphilales, Methylophilales, Neisseriales, Nitrosomonadales, Procabacteriales* oder *Rhodocyclales.*. Ganz besonders bevorzugt aus Bakterien der Familie der *Rhodocyclaceae.* Insbesondere bevorzugt aus den Gattung *Azoarcus.* Insbesondere bevorzugt aus Arten *Azoarcus anaerobius, Azoarcus buckelii, Azoarcus communis, Azoarcus evansii, Azoarcus indigens, Azoarcus toluclasticus, Azoarcus tolulyticus, Azoarcus toluvorens, Azoarcus* sp., *Azoarcus* sp. 22Lin, *Azoarcus* sp. BH72, *Azoarcus* sp. CC-11, *Azoarcus* sp. CIB, *Azoarcus* sp. CR23, *Azoarcus* sp. EB1, *Azoarcus* sp. EbN1, *Azoarcus* sp. FL05, *Azoarcus* sp. HA, *Azoarcus* sp. HxN1, *Azoarcus* sp. mXyN1, *Azoarcus* sp. PbN1, *Azoarcus* sp. PH002, *Azoarcus* sp. T und *Azoarcus* sp. ToN1.

Besonders bevorzugt verwendet man Dehydrogenasen aus *Azoarcus* sp EbN1.

Nukleinsäuresequenzen lassen sich beispielsweise mit üblichen Hybridisierungsverfahren oder der PCR-Technik aus anderen Organismen, z.B. über genomische oder cDNA-Banken, isolieren. Diese DNA-Sequenzen hybridisieren unter Standardbedingungen mit den Sequenzen. Zur Hybridisierung werden vorteilhaft kurze Oligonukleotide der konservierten Bereiche beispielsweise aus dem aktiven Zentrum, die über Vergleiche mit einer Dehydrogenase in dem Fachmann bekannter Weise ermittelt werden können, verwendet. Es können aber auch längere Fragmente der Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Gegenstand der Beschreibung sind auch Derivate der konkret offenbarten oder ableitbaren Nukleinsäuresequenzen.

So können weitere Nukleinsäuresequenzen von Seq ID 1 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Beschrieben sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Gegenstand der Beschreibung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten einer Nukleinsäuresequenz sind beispielsweise Allelvarianten zu verstehen, die mindestens

40 % Homologie auf der abgeleiteten Aminosäureebene, bevorzugt mindestens 60 % Homologie, ganz besonders bevorzugt mindestens 80, 85, 90, 93, 95 oder 98 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der Nukleinsäuresequenzen beispielsweise pilzliche oder bakterielle Homologe, verkürzte Sequenzen, Einzelstrang-DNA oder RNA der kodierenden und nichtkodierenden DNA-Sequenz, zu verstehen. So besitzen z.B. auf DNA-Ebene eine Homologie von mindestens 40 %, bevorzugt von mindestens 60 %, besonders bevorzugt von mindestens 70 %, ganz besonders bevorzugt von mindestens 80 % über den gesamten angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch ein oder mehrere Nukleotidaustausche, Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Unter Derivaten sind auch Varianten zu verstehen, deren Nukleotidsequenz im Bereich von -1 bis -1000 Basen stromaufwärts vor dem Startkodon oder 0 bis 1000 Basen stromabwärts nach dem Stoppkodon so verändert wurden, dass die Genexpression und/oder die Proteinexpression verändert, bevorzugt erhöht wird.

Weiterhin umfasst die Beschreibung auch Nukleinsäuresequenzen, welchen mit oben genannten kodierenden Sequenzen unter "stringenten Bedingungen" hybridisieren. Diese Polynukleotide lassen sich bei der Durchmusterung von genomischen oder cDNA-Banken auffinden und gegebenenfalls daraus mit geeigneten Primern mittels PCR vermehren und anschließend beispielsweise mit geeigneten Sonden isolieren. Darüber hinaus können Polynukleotide auch auf chemischem Wege synthetisiert werden. Unter dieser Eigenschaft versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen zu 70-100%, vorzugsweise zu 90-100%, komplementär sein. Die Eigenschaft komplementärer Sequenzen, spezifisch aneinander binden zu können, macht man sich beispielsweise in der Northern- oder Southem-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 - 70°C, vorzugsweise 60 - 65°C warmen Waschlösung, beispielsweise 0,1x SSC-Puffer mit 0,1% SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z.B. in Ausubel et al., Current Protocols in Molecular Biology, John Wiley & Sons, N.Y. (1989), 6.3.1-6.3.6. beschrieben.

Ausgestaltungen der Konstrukte

Gegenstand der Beschreibung sind außerdem Expressionskonstrukte, enthaltend unter der genetischen Kontrolle regulativer ukleinsäuresequenzen eine für ein Polypeptid kodierende Nukleinsäuresequenz; sowie Vektoren, umfassend wenigstens eines dieser Expressionskonstrukte.

Vorzugsweise umfassen solche Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185. Academic Press, San Diego, CA (1990). Unter einem Nukleinsäurekonstrukt sind insbesondere solche zu verstehen, bei weichen das Gen für eine Dehydrogenase mit einem oder mehreren Regulationssignalen zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten

"Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Vorteilhafte Regulationssequenzen für das erfindungsgemäße Verfahren sind beispielsweise in Promotoren wie cos-, tac-, trp-, tet-, trp-tet-, lpp-, lac-, lpp-lac-, $lacI^{q-}$, T7-, T5-, T3-, gal-, trc-, ara-, rhaP ($rhaP_{BAD}$)SP6-, lambda-$P_R$- oder im lambda-$P_L$-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. In diesem Zusammenhang sind auch die Promotoren der Pyruvatdecarboxylase und der Methanoloxidase, beispielsweiseaus *Hansenula* vorteilhaft. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Beschreibung dar. Geeignete Plasmide sind beispielsweise in *E. coli* pLG338, pACYC184, p8R322, pUC18, pUC19, pKC30, pRep4, pHS1, pKK223-3, pDHE19.2, pHS2, pPLc236, pMBL24, pLG200, pUR290, pIN-$III^{113}$-B1, λgt11 oder pEdCl, in *Streptomyces* pIJ101, pIJ364, pIJ702 oder pIJ361, in *Bacillus* pUB110, pC194 oder pBD214, in *Corynebacterium* pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, $pGHlac^{-}$, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985, ISBN 0 444 904018) entnommen werden.

Vorteilhafterweise enthält das Nukleinsäurekonstrukt zur Expression der weiteren enthaltenen Gene zusätzlich noch 3'- und/oder 5'-terminale regulatorische Sequenzen zur Steigerung der Expression, die je nach ausgewähltem Wirtorganismus und Gen oder Gene für eine optimale Expression ausgewählt werden.

Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

In einer weiteren Ausgestaltungsform des Vektors kann der das Nukleinsäurekonstrukt oder die Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der Nukleinsäure bestehen.

Für eine optimale Expression heterologer Gene in Organismen ist es vorteilhaft die Nukleinsäuresequenzen entsprechend des im Organismus verwendeten spezifischen "codon usage" zu verändern. Der "codon usage" lässt sich anhand von Computerauswertungen anderer, bekannter Gene des betreffenden Organismus leicht ermitteln.

Die Herstellung einer Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

Branchbare Wirtsorganismen

Mit Hilfe der Vektoren oder Konstrukte sind rekombinante Mikroorganismen herstellbar, welche beispielsweise mit wenigstens einem Vektor transformiert sind und zur Produktion der Polypeptide eingesetzt werden können. Vorteilhafterweise werden die oben beschriebenen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory. Cold Spring Harbor Laboratory Press, Cold Spring Harbor, NY, 1989 beschrieben.

Es sind auch homolog rekombinierte Mikroorganismen herstellbar. Dazu wird ein Vektor hergestellt, der zumindest einen Abschnitt eines Gens oder einer kodierenden Sequenz enthält, worin gegebenenfalls wenigstens eine Aminosäure-Deletion, - Addition oder-Substitution eingebracht worden ist, um die Sequenz zu verändern, z.B. funktionell zu disrumpieren ("Knockout"-Vektor). Die eingebrachte Sequenz kann z.B. auch ein Homologes aus einem verwandten Mikroorganismus sein oder aus einer Säugetier-, Hefe- oder Insektenquelle abgeleitet sein. Der zur homologen Rekombination verwendete Vektor kann alternativ derart ausgestaltet sein, daß das endogene Gen bei homologer Rekombination mutiert oder anderweitig verändert ist, jedoch noch das funktionelle Protein kodiert (z.B. kann der stromaufwärts gelegene regulatorische Bereich derart verändert sein, dass dadurch die Expression des endogenen Proteins verändert wird). Der veränderte Abschnitt des Gens ist im homologen Rekombinationsvektor. Die Konstruktion geeigneter Vektoren zur homologen Rekombination ist z.B. beschrieben in Thomas, K.R. und Capecchi, M.R. (1987) Cell 51:503.

Als rekombinante Wirtsorganismen für die Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien *Enterobactedaceae, Pseudomonadaceae, Rhizobiaceae, Streptomycetaceae* oder Nocardiaceae, besonders bevorzugt Bakterien der Gattungen *Escherichia, Pseudomonas, Streptomyces, Nocardia, Burkholderia, Salmonella, Agrobacterium* oder *Rhodococcus* verwendet. Ganz besonders bevorzugt ist die Gattung und Art *Escherichia coli.* Weitere vorteilhafte Bakterien sind darüber hinaus in der Gruppe der α-Proteobakterien, β-Proteobakterien oder γ-Proteobakterien zu finden.

Der Wirtsorganismus oder die Wirtsorganismen gemäß der Beschreibung enthalten dabei vorzugsweise mindestens eine der in dieser Beschreibung beschriebenen Nukleinsäuresequenzen, Nukleinsäurekonstrukte oder Vektoren, die für ein Enzym mit Dehydrogenaseaktivität kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden. Das Keton kann direkt zur Anzucht gegeben werden oder vorteilhaft nach Anzucht. Die Enzyme können nach dem in den Beispielen beschriebenen Verfahren aus den Organismen isoliert werden oder als Rohextrakt für die Reaktion verwendet werden.

Rekombinante Herstellung der Polypeptide

Gegenstand der Beschreibung sind weiterhin Verfahren zur rekombinanten Herstellung der Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Der rekombinante Mikroorganismus kann nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Die Zellen werden dann, falls die Polypeptide nicht in das Kulturmedium sezerniert werden, ausgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch

Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

Weitere Ausgestaltungen zur Durchführung des erfindungsgemäßen enzymatischen Reduktionsverfahrens

Die Dehydrogenasen können im erfindungsgemäßen Verfahren als freies oder immobilisiertes Enzym oder als noch im rekombinanten Produktionsorganismus enthaltenen Katalysator verwendet werden.

Das erfindungsgemäße Verfahren wird vorteilhaft bei einer Temperatur zwischen 0 °C bis 95 °C, bevorzugt zwischen 10 °C bis 85 °C, besonders bevorzugt zwischen 15 °C bis 75 °C durchgeführt.

Der pH-Wert im erfindungsgemäßen Verfahren wird vorteilhaft zwischen pH 4 und 12, bevorzugt zwischen pH 4,5 und 9, besonders bevorzugt zwischen pH 5 und 8 gehalten.

Unter enantiomerenreinen bzw. chiralen Produkten sind im erfindungsgemäßen Verfahren Enantiomere zu verstehen, die eine Enantiomerenanreicherung zeigen. Bevorzugt werden im Verfahren Enantiomerenreinheiten von mindestens 70 %ee, bevorzugt von min. 80 %ee, besonders bevorzugt von min. 90 %ee, ganz besonders bevorzugt min. 98 %ee erreicht.

Für das erfindungsgemäße Verfahren können wachsende Zellen verwendet werden, die die Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Das erfindungsgemäße Verfahren kann batchweise, semi-batchweise oder kontinuierlich betrieben werden.

Die Durchführung des Verfahrens kann vorteilhafterweise in Bioreaktoren erfolgen, wie z.B. beschrieben in Biotechnology, Band 3, 2. Auflage, Rehm et al Hrsg., (1993) insbesondere Kapitel II.

Die nachfolgenden Beispiele sollen die Erfindung veranschaulichen.

Experimenteller Teil

Vergleichendes Beispiel 1: Klonierung der Alkohol-Dehydrogenase EbN2 aus *Azoarcus* sp. EbN1.

Die Sequenz des Dehydrogenasegens EbN2 aus *Azoarcus* sp. EbN1 ist in Datenbanken hinterlegt (Seq ID 1, [Genbank ID 56475432, Region: 2797788..2798528]). Von der Nukleinsäuresequenz des Gens wurden Oligonukleotide abgeleitet, mit denen nach bekannten Verfahren das Gen aus genomischer DNA von *Azoarcus* sp. EbN1 amplifiziert wurde. Die erhaltene Sequenz entspricht der publizierten Sequenz. Die DNA-Sequenz der Oligonukleotide ist in Tabelle 1 zusammengefasst.

PCR-Bedingungen:

| | |
|---|---|
| 2µL | 10*Pfu-Ultra Puffer (Stratagene) |
| 100 ng | Primer#1 (vgl. Tabelle 1) |
| 100 ng | Primer#2 (vgl. Tabelle 1) |
| 1 µL | dNTP (je 10 mM) |
| ca. 30 ng | chromosomale DNA aus *Azoarcus sp. EbN1* |
| 1 U | *Pfu*-Ultra DNA Polymerase |
| ad 20 µL | $H_2O$ |

Temperaturprogramm:

5 min, 94°C,
60 sec, 50°C, ⎫
2 min, 72°C, ⎬ (35 Zyklen)
60 sec, 94°C, ⎭
10 min, 72°C,
∞, 10°C

Das PCR-Produkt (ca. 751 bp) wurde mit den Restriktionsendonukleasen *Nde*I und *Bam*HI verdaut und in entsprechend verdauten pDHE19.2-Vektor (DE19848129) kloniert. Die Ligationsansätze wurden in E.coli XL1 Blue (Stratagene) transformiert.
Das erhaltene Plasmid pDHE-PDH-L wurde in den Stamm *E.coli* TG10 pAgro4 pHSG575 transformiert (TG10: ein RhaA-Derivat von E.coli TG1(Stratagene): pAgro4: Takeshita, S; Sato, M; Toba, M; Masahashi, W; Hashimoto-Gotoh, T (1987) Gene 61, 63-74; pHSG575: T. Tomoyasu et al (2001), Mol. Microbiol. 40(2), 397-413).
Die rekombinanten *E. coli* werden mit LU 13151 bezeichnet.

Vergleichendes Beispiel 2: Klonierung der Alkohol-Dehydrogenase ChnA aus *Azoarcus* sp. EbN1.

Die Sequenz des Dehydrogenasegens ChnA aus *Azoarcus* sp. EbN1 ist in Datenbanken hinterlegt ([Genbank ID 56475432, Region: (complement) 192247..192993]). Von der Nukleinsäuresequenz des Gens wurden Oligonukleotide abgeleitet, mit denen nach bekannten Verfahren das Gen aus genomischer DNA von *Azoarcus* sp. EbN1 amplifiziert wurde. Die erhaltene Sequenz entspricht der publizierten Sequenz. Die DNA-Sequenz der Oligonukleotide ist in Tabelle 2 zusammengefasst.

PCR-Bedingungen:

| | |
|---|---|
| 2 µL | 10*Pfu-Ultra Puffer (Stratagene) |
| 100 ng | Primer #3 (vgl.Tabelle 2) |
| 100 ng | Primer #4 (vgl. Tabelle 2) |
| 1 µL | dNTP (je 10 mM) |
| ca. 30 ng | chromosomale DNA aus *Azoarcus sp. EbN1* |
| 1 U | *Pfu*-Ultra DNA Polymerase |
| ad 20 µL | $H_2O$ |

Temperaturprogramm:

5 min, 94°C,
60 sec, 50°C,
2 min, 72°C, } (35 Zyklen)
60 sec, 94°C,
10 min, 72°C,
∞, 10°C

Das PCR-Produkt (ca. 743bp) wurde mit den Restriktionsendonukleasen *Nde*I und *Bgl*II verdaut und in einen *Nde*I und *Bam*HI restringierten pDHE19.2-Vektor (DE19848129) kloniert. Die Ligationsansätze wurden in *E.coli* XL1 Blue (Stratagene) transformiert.

Das erhaltene Plasmid *p*DHE-PDH-L wurde in den Stamm *E.coli* TG10 pAgro4 pHSG575 transformiert (TG10: ein $RhaA^-$-Derivat von E.coli TG1(Stratagene); pAgro4: Takeshita, S; Sato, M; Toba, M; Masahashi, W; Hashimoto-Gotoh, T (1987) Gene 61, 63-74; pHSG575: T. Tomoyasu et al (2001), Mol. Microbiol. 40(2), 397-413).

Die rekombianten *E. coli* werden mit LU 13283 bezeichnet.

Vergleichendes Beispiel 3: Bereitstellung rekombinanter 'anti-Prelog'-Dehydrogenasen

LU 13151 oder LU 13283 wurden in 20mL LB-Amp/Spec/Cm (100μg/l Ampicillin; 100μg/l Spectinomycin; 20μg/l Chloramphenicol), 0,1mM IPTG, 0,5g/L Rhamnose in 100mL Erlenmeyerkolben (Schikanen) 18 h bei 37°C angezogen, bei 5000*g/10min zentrifugiert, einmal mit 10mM TRIS*HCl, pH7,0 gewaschen und in 2 mL des gleichen Puffers resuspendiert.

Zellfreier Proteinrohextrakt wurde hergestellt indem Zellpaste von LU 13151 bzw. LU 13283 0,7ml Glaskugeln (d=0,5mm) in einer Schwingmühle (3x 5min mit Zwischenkühlung auf Eis) aufgeschlossen wurde.

Vergleichendes Beispiel 4: Aktivitätsbestimmung der rekombinanten 'anti-Prelog'-Dehydrogenasen aus *Azoarcus* sp. EbN1

Je 6 Transformanten wurden in 20mL LB Amp/Spec/Cm (100μg/l Amp; 100mg/lSpec; 20μg/lCm) 0,1mM IPTG 0,5g/L Rhamnose in 100mL Erlenmeyerkolben (Schikanen) 18 h bei 37°C angezogen, bei 5000*g/10min zentrifugiert, einmal mit 10mM Tris/HCl pH7,0 gewaschen und in 2 mL des gleichen Puffers resuspendiert.

Zellfreier Rohextrakt der rekombinanten *E.coli*, die die Dehydrogenasegene enthalten wurde durch Zellaufschluss mit 0,7ml Glaskugeln (d=0,5mm) in einer Schwingmühle (3x 5min mit Zwischenkühlung auf Eis) gewonnen.

Im Photometer kann bei 340 nm der Verbrauch reduzierter Kosubstrate während der Reduktion von Ketonen verfolgt werden. In 1 mL 50 mM $KP_i$, 1 mM $MgCl_2$, pH 6.5 wurden bei 30° C 10 μL verdünnter zellfreier Rohextrakt (≡ 10 μg Protein), 10 μmol Keton und 250 nmol NADH oder NADPH inkubiert. 1 Unit (1 U) entspricht der Enzymmenge, die in 1 min 1μmol Keton reduziert.

Vergleichendes Beispiel 5 Analytik von Phenylethanol

Die Konzentration von Acetophenon und Phenylethanol lassen sich mittels HPLC bestimmen. Je nach Wahl der stationären und mobilen Phase lassen sich neben der Konzentration auch ee-Wert bestimmen.

| | |
|---|---|
| stationäre Phase: | Hydrodex ß-6-TBDM (Macherey&Nagel), Länge: 25m, ∅: 250μm, |
| mobilePhase: | Helium, split 100:1, Gesamtfluss: 92mL/min, Druck: 17 psi |
| Flussrate: | 1.0 ml/min |
| Detektion: | FID |
| Temperaturgradient: | t=0min: 90°C, Heizen mit 3°/min auf 140°C |
| Detektortemperatur: | 250°C |
| Retentionszeiten: | Acetophenon: ca. 7,5 min |
| | (1*S*)-Phenylethanol: ca. 12,5 min |
| | (1*R*)-Phenylethanol: ca. 12,1 min |

Mit authentischem Material wird eine Eichreihe erstellt, anhand derer die Konzentration unbekannter Proben

bestimmt werden kann.

Beispiel 6: Bereitstellung von Glukose-Dehydrogenase zur Kofaktor-Regenerierung und Kofaktorregenerierung mit Glukosedehydrogenase (Enzymkopplung)

Zur Kofaktor-Regenerierung kann Glukose-Dehydrogenase verwendet werden. Das Enzym ist aus kommerziellen (z.B. Jülich Fine Chemicals Order-No. 22.10 oder 19.10) oder eigenen Quellen zugänglich. Bei letzterem handelt es sich um ein *E. coli* XL10 Gold Klon der im Plasmid pUC19 das Glukose-Dehydrogenase-Gen aus *Bacillus subtilis* (Genbank-Acc.No. M12276) enthält (Dieses Konstrukt trägt die Bezeichnung *E. coli* LU11293.

Zur Fermentation von *E. coli* LU11293 wurde folgendes Medium angesetzt:

| | |
|---|---|
| 560 g | Hefeextrakt (65 %) |
| 448 g | Trypton (Difco) |
| 42 g | $KH_2PO_4$ |
| 84 g | $Na_2HPO_4$ |
| 644 g | Glycerin (99%) |
| 100 mL | SL4 Lösung (5 fach) |
| | |
| 1 g | Tegosipon 3062 |
| | Medium mit Wasser auf 13,5 L auffüllen, pH-Wert |
| | auf 7,0 einstellen, ca. 300 mL für Vorkultur ent- |
| | nehmen, danach 30 min. bei 122 °C sterilisieren. |
| | |
| | Sterile Salzlösung* zugeben (vorher die Salzlösung für |
| | die Schüttelkolben entnehmen, siehe Rapport). |
| *Salzlösung: 2,1 g $CaCl_2$ * 2 $H_2O$<br>3,5 g $MgSO_4$ * 7 $H_2O$<br>14 g $NH_4Cl$<br>14 mL Ampicillin-Lösung (100 mg/mL)<br>in 500 mL Wasser lösen und sterilfiltrieren | |

Jeweils 150 mL Medium wurden in zwei 1 L Erlenmeyerkolben sterilisiert und mit 5 mL steriler Salzlösung komplettiert. Nach Beimpfen von einer LB-Ampicillin-Agarplatte wurden die Vorkulturen 12 Stunden bei 37 °C und 200 UPM inkubiert und zum Fermentationsmedium gegeben. Die Fermentation wurde bei 37°C, 0,1 bar Innendruck, pH 7,0 (Regelung mit 20% Phosphorsäure und 25% NaOH) mit einer Begasungsrate von 7,5 L/min und 300 upm gestartet (Regelung $pO_2$ zwischen 20 und 50% mit 10-20 Umin Zuluft und 500-1500 Upm). Nach 2 h wurden zur Induktion 0,1 mM IPTG zugegeben und nach insgesamt 13 h die Fermentation beendet. Nach Ernte und Waschen der Zellen (1,3 kg) wurden diese bis zur Verwendung (2-20 g/L im Ansatz) bei -20°C gelagert.

Equimolare Mengen Glukose und Keton werden mit 1-30U/mL Glukosedehydrogenase-Rohextrakt und 1-30U/mL, Alkoholdehydrogenase-Rohextrakt 0.02-1 mmol/L NAD bzw. NADP oder NADH bzw. NADPH wurden in Puffer gelöst und bei 10-60°C inkubiert. Der pH-Wert wurde durch automatische Zugabe von Base konstant gehalten.

Vergleichendes Beispiel 7: Kofaktorregenerierung mit Substratkopplung

Die Regenerierung des Kofaktors kann auch durch die beiden Alkoholdehydrogenasen selbst durchgeführt werden. In diesem Fall ist die Zugabe eines gesonderten Regenerationsenzyms nicht notwendig. Die Alkoholdehydrogenase ChnA und Ebn2 akzeptierten verschiedene einfache Alkohole als Reduktionsmittel. Sie werden zu den entsprechenden Carbonylverbindungen oxidiert. Einfache Alkohole, die sich zur Regeneration von NADH oder NADPH mit eignen, sind iso-Propanol, Butan-2-ol und Pentan-2-ol.

Vergleichendes Beispiel 8: Kofaktorregenerierung mit Formiat-Dehydrogenase (Enzymkopplung)

Zur Kofaktorregenerierung kann Formiat-Dehydrogenase verwendet werden. Das Enzym ist aus kommerziellen (z.B. Jülich Fine Chemicals Order-No. 09.11, 24.11 oder 25.10) oder aus eigenen Quellen zugänglich. Analog Beispiel 6 kann somit die Regenerierung der Kofaktoren auch mit Formiat-Dehydrogenase erfolgen. Dabei werden equimolare Mengen Formiat und Keton mit 1-30U/mL Formiat-Dehydrogenase-Rohextrakt und 1-30U/mL, Alkoholdehydrogenase-Rohextrakt 0.02-1 mmol/L NAD bzw. NADP oder NADH bzw. NADPH wurden in Puffer gelöst und bei 10-60°C inkubiert. Der pH-Wert wurde durch automatische Zugabe von Säure konstant gehalten.

Beispiel 9: Herstellung von *R*-Phenylethanol mit rekombinanten *anti*-Prelog-Dehydrogenasen *E.coli* LU 13151 und vergleichend LU 13283 wurden entsprechend Beispiel 3 angezogen, geerntet und aufgeschlossen.

Pro Liter Reaktionsvolumen werden 0,2mmol NAD, 500U Glukosedehydrogenase, 1mol D-Glukose, 1 mol Acetophenon, 100-1000 U Alkoholdehydrogenase aus LU 13283 oder LU 13151 in $KP_i$-Puffer (50mM $KP_i$, 1mM $MgCl_2$, pH 6.5) gelöst und bei 30°C inkubiert. Der pH-Wert wurde durch automatische Zugabe von 2M NaOH konstant gehalten. Es ist durchaus plausibel, dass höhere Endkonzentrationen an *R*-Phenylethanol erreicht werden können, wenn Acetophenon im Verlauf der Reaktion nachdosiert wird (fed-batch-Fahrweise).
Ebenso ist eine Reaktion in Gegenwart organischer, nicht wasserlöslicher Lösungsmittel möglich.

**Patentansprüche**

1. Verfahren zur Herstellung von optisch aktiven Alkoholen der Formel Ia oder Ib

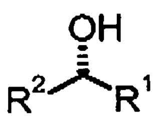

Formel Ia

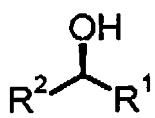

Formel Ib

worin

$R^1$ ,$R^2$ für Alkyl, Alkenyl, Aryl, oderAlkylarylgruppen stehen, die wiederum ein- oder mehrfach substituiert sein können, durch Alkyl, Halogen, SH,$SR^2$ ,OH, $OR^2$, $NO_2$, CN, CO, $COOR^2$, $NR^2R^3$ oder $NR^2R^3R^{4+}X$, wobei $R^2$, $R^3$ und $R^4$ unabhängig voneinander für H oder einen Alkyl- oder Alkoxy-Rest mit 1-6 C-Atomen stehen und $X^-$ für ein Gegenion steht, mit der Maßgabe, dass $R^1$ungleich $R^2$ ist,

durch Reduktion des entsprechenden Ketons, wobei die Reduktion mit einer Dehydrogenase mit der Polypeptidsequenz SEQ ID NO:2 oder mit einer Polypeptidsequenz, bei der bis zu 25% der Aminosäurereste gegenüber SEQ ID NO:2 durch Deletion, Insertion, Substitution oder einer Kombination davon verändert sind, durchgeführt wird.

**2.** Verfahren nach Anspruch 1, **dadurch gekennzeichnet, dass** die Dehydrogenase in einem Wirtsorganismus rekombinant exprimiert wird und die Reaktionslösung mit diesem Wirtsorganismus inkubiert wird.

**3.** Verfahren nach Anspruch 2, **dadurch gekennzeichnet, dass** der Wirtsorganismus zuvor abgetötet worden ist.

**4.** Verfahren nach Anspruch 1, **dadurch gekennzeichnet, dass** die Reduktion bei einer Temperatur von 20-40°C durchgeführt wird.

**5.** Verfahren nach Anspruch 1, **dadurch gekennzeichnet, dass** der reduzierte Cofaktor durch das Enzym selber regeneriert wird oder dass als Cofaktor regenerierendes System Glucosedehydrogenase, Phosphitdehydrogenase, Formiatdehydrogenase oder eine andere Alkoholdehydrogenase verwendet wird.

## Claims

**1.** A process for preparing optically active alcohols of the formula Ia or Ib

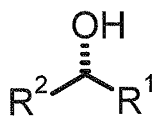

Formula Ia

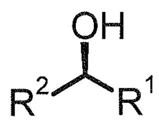

Formula Ib

in which

$R^1$, $R^2$ are alkyl, alkenyl, aryl, or alkylaryl groups which may in turn be substituted one or more times by alkyl, halogen, SH, $SR^2$, OH, $OR^2$, $NO_2$, CN, CO, $COOR^2$, $NR^2R^3$ or $NR^2R^3R^{4+}X$, where $R^2$, $R^3$ and $R^4$ are independently of one another H or an alkyl or alkoxy radical having 1-6 carbon atoms, and $X^-$ is a counter ion, with the proviso that $R^1$ is not equal to $R^2$,

by reducing the corresponding ketone, where the reduction is carried out with a dehydrogenase having the polypeptide sequence of SEQ ID NO: 2, or with a polypeptide sequence in which up to 25% of the amino acid residues are altered by comparison with SEQ ID NO: 2 by deletion, insertion, substitution or a combination thereof.

**2.** The process according to claim 1, wherein the dehydrogenase is expressed recombinantly in a host organism, and the reaction solution is incubated with this host organism.

**3.** The process according to claim 2, wherein the host organism has previously been killed.

**4.** The process according to claim 1, wherein the reduction is carried out at a temperature of 20-40°C.

**5.** The process according to claim 1, wherein the reduced cofactor is regenerated by the enzyme itself, or wherein glucose dehydrogenase, phosphite dehydrogenase, formate dehydrogenase or another alcohol dehydrogenase is used as cofactor-regenerating system.

## Revendications

1. Procédé de préparation d'alcools optiquement actifs de formule Ia ou Ib

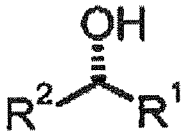

Formule Ia

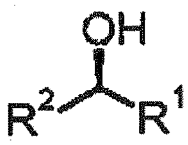

Formule Ib

dans laquelle

$R^1$, $R^2$ représentent des groupes alkyle, alcényle, aryle ou alkylaryle, qui pour leur part peuvent être une ou plusieurs fois substitués par un ou des substituants alkyle, halogéno, SH, $SR^2$, OH, $OR^2$, $NO_2$, CN, CO, $COOR^2$, $NR^2R^3$ ou $NR^2R^3R^{4+}X$, où $R^2$, $R^3$ et $R^4$ représentent chacun indépendamment des autres H ou un radical alkyle ou alcoxy ayant 1 à 6 atomes de carbone, et X est un contre-ion, à la condition que $R^1$ soit différent de $R^2$,

par réduction de la cétone correspondante, la réduction étant mise en oeuvre avec une déshydrogénase ayant la séquence polypeptidique SEQ ID N° 2 ou une séquence polypeptidique dans laquelle jusqu'à 25 % des résidus d'acides aminés sont modifiés par rapport à SEQ ID N° 2 par délétion, insertion, substitution ou une combinaison de celles-ci.

2. Procédé selon la revendication 1, **caractérisé en ce que** la déshydrogénase est soumise à une expression par recombinaison dans un organisme hôte, et la solution réactionnelle est incubée avec cet organisme hôte.

3. Procédé selon la revendication 2, **caractérisé en ce que** l'organisme hôte a été au préalable tué.

4. Procédé selon la revendication 1, **caractérisé en ce que** la réduction est mise en oeuvre à une température de 20 à 40°C.

5. Procédé selon la revendication 1, **caractérisé en ce que** le co-facteur réduit est régénéré par l'enzyme proprement dite, ou que l'on utilise en tant que système régénérateur de co-facteurs la glucose déshydrogénase, la phosphite déshydrogénase, la formiate déshydrogénase ou une autre alcool déshydrogénase.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2005108590 A
- WO 2006094945 A
- EP 1149849 A
- EP 1069183 A
- DE OS100193773 A
- DE 19848129


### In der Beschreibung aufgeführte Nicht-Patentliteratur


- *Chemico-Biological Interactions,* 2003, vol. 143, 247
- *Journal of Biological Chemistry,* 2002, vol. 277, 25677
- *Tetrahedron,* 2004, vol. 60, 633
- *Trends Biotechnol,* 1999, vol. 17, 487
- *Pure and Applied Chemistry,* 1964, vol. 9, 119
- *J.Org.Chem.,* 1992, vol. 57, 1532
- **PRELOG, V.** *Pure and Applied Chemistry,* 1964, vol. 9, 119-130
- Reduction of Ketones. **K. NAKAMURA ; T. MATSUDA.** Enzyme Catalysis in Organic Synthesis. Wiley-VCH, 2002, vol. III, 991-1032
- **W. HUMMEL ; K. ABOKITSE ; K. DRAUZ ; C. ROLLMANN ; H. GRÖGER.** *Adv. Synth. Catal.,* 2003, vol. 345 (1 + 2), 153-159
- Oxidation of Alcohols. **A. SCHMIDT ; F. HOLLMANN ; B. BÜHLER.** Enzyme Catalysis in Organic Synthesis. Wiley-VCH, 2002, vol. III, 991-1032
- Immobilization of Enzymes. **J. LALONDE ; A. MARGOLIN.** Enzyme Catalysis in Organic Synthesis. Wiley-VCH, 2002, vol. III, 991-1032
- **PEARSON ; LIPMAN.** *Proc. Natl. Acad, Sci. (USA,* 1988, vol. 85 (8), 2444-2448
- **NARANG, S.A.** *Tetrahedron,* 1983, vol. 39, 3
- **ITAKURA et al.** *Annu. Rev. Biochem.,* 1984, vol. 53, 323
- **ITAKURA et al.** *Science,* 1984, vol. 198, 1056
- **IKE et al.** *Nucleic Acids Res.,* 1983, vol. 11, 477
- **ARKIN ; YOURVAN.** *PNAS,* 1992, vol. 89, 7811-7815
- **DELGRAVE et al.** *Protein Engineering,* 1993, vol. 6 (3), 327-331
- **SAMBROOK et al.** Molecular Cloning: A laboratory manual. Cold Spring Harbor Laboratory Press, 1989
- **SAMBROOK, J. ; FRITSCH, E.F. ; MANIATIS, T.** Molecular Cloning: A Laboratory Manual. Cold Spring Harbor Laboratory Press, 1989
- **SAMBROOK et al.** Molecular Cloning. Cold Spring Harbor Laboratory, 1989
- Current Protocols in Molecular Biology. John Wiley & Sons, 1985
- Nucleic Acids Hybridization: A Practical Approach. IRL Press at Oxford University Press, 1985
- Essential Molecular Biology: A Practical Approach. IRL Press at Oxford University Press, 1991
- **AUSUBEL et al.** Current Protocols in Molecular Biology. John Wiley & Sons, 1989, 6.3.1-6.3.6
- **GOEDDEL.** Gene Expression Technology: Methods in Enzymology. Academic Press, 1990, vol. 185
- Buch Cloning Vectors. Elsevier, 1985
- **T. MANIATIS ; E.F. FRITSCH ; J. SAMBROOK.** Molecular Cloning: A Laboratory Manual. Cold Spring Harbor Laboratory, 1989
- **T.J. SILHAVY ; M.L. BERMAN ; L.W. ENQUIST.** Experiments with Gene Fusions. Cold Spring Harbor Laboratory, 1984
- **AUSUBEL, F.M. et al.** Current Protocols in Molecular Biology. Greene Publishing Assoc. and Wiley Interscience, 1987
- Cloning Vectors. Elsevier, 1985
- Current Protocols in Molecular Biology. Wiley Interscience, 1997
- **SAMBROOK et al.** Molecular Cloning: A Laboratory Manual. Cold Spring Harbor Laboratory Press, 1989
- **THOMAS, K.R. ; CAPECCHI, M.R.** *Cell,* 1987, vol. 51, 503
- **COOPER, F. G.** Biochemische Arbeitsmethoden. Verlag Walter de Gruyter
- **SCOPES, R.** Protein Purification. Springer Verlag
- **HARLOW, E. ; LANE, D.** Antibodies: A Laboratory Manual. Cold Spring Harbor (N.Y.) Press, 1988
- Biotechnology. 1993, vol. 3
- **TAKESHITA, S ; SATO, M ; TOBA, M ; MASAHASHI, W ; HASHIMOTO-GOTOH, T.** *Gene,* 1987, vol. 61, 63-74

- **T. TOMOYASU et al.** *Mol. Microbiol.,* 2001, vol. 40 (2), 397-413